# EUROPEAN PATENT APPLICATION

(11) **EP 4 628 896 A1**
(43) Date of publication of application: **08.10.2025**
(21) Application number: 23898240.9
(22) Date of filing: 27.11.2023
(51) Int. Cl.: G01N 33/68, C12Q 1/6883

(54) **BIOMARKER FOR DIAGNOSING SARCOPENIA AND SARCOPENIA DIAGNOSIS METHOD USING SAME**

(30) Priority: 28.11.2022 KR 20220161823
(71) Applicant: The Asan Foundation, Seoul 05505 (KR); UNIVERSITY OF ULSAN FOUNDATION FOR INDUSTRY COOPERATION, Nam-gu Ulsan 44610 (KR)
(72) Inventor: KIM, Kyung Gon, Seoul 05507 (KR); KIM, Beom Jun, Seoul 06310 (KR)
(74) Representative: Dehns
(86) International application number: PCT/KR2023/019243
(87) International publication number: WO 2024/117714

(57) **Abstract**

The present invention relates to a composition for diagnosing sarcopenia, and the like, and was completed by discovering biomarkers enabling the diagnosis of sarcopenia with high accuracy and sensitivity through qualitative and quantitative analysis of the proteome of a sarcopenia animal model. In particular, the biomarkers according to the present invention were confirmed to have a positive or negative correlation with sarcopenia-related indicators such as muscle mass, grip strength, rotarod score, and inverted strength. Therefore, instead or in addition to conventional methods that have relied on the measurement of muscle mass and muscle function, through molecular diagnosis using the markers of the present invention, it is possible to diagnose sarcopenia, and furthermore, it is also possible to determine the severity of sarcopenia through the association of the markers with muscle function. In particular, biomarkers which show consistent correlations with sarcopenia indicators are also expected to be applicable as targets for the treatment of sarcopenia. Furthermore, since sarcopenia is a disease closely related to aging, the biomarkers according to the present invention are expected to be the basis for securing of anti-aging treatment targets and the development of aging control technologies.

## Description

### [Technical Field]

The present invention relates to a biomarker for diagnosing sarcopenia and a sarcopenia diagnosis method using the same.

This application claims priority to and the benefit of Korean Patent Application No. 10-2022-0161823, filed on November 28, 2022, the disclosure of which is incorporated herein by reference in its entirety.

### [Background Art]

Sarcopenia is a disease in which muscle strength and physical function decline due to a decrease in skeletal muscle mass. It is an official disease that was given a diagnosis code in the United States (ICD-10-CM) in 2016 and in Korea (KCD-8) in 2021. There are various causes of sarcopenia, but the most common are reduced protein intake and lack of exercise. In particular, the rate of sarcopenia due to insufficient intake and absorption of essential amino acids is very high. Another common cause of sarcopenia is hormonal deficiency accompanying aging. Skeletal muscle is the largest organ in the human body, accounting for 40% to 50% of the human body. Skeletal muscle tends to decrease by about 1% per year after the age of 30, and then decreases rapidly after the age of 65. As aging progresses, the functional capacity of muscles also gradually decreases, and muscle mass decreasing along with muscle strength and muscle function is diagnosed as sarcopenia.

Muscles are not only very important as a motor organ, but also affect the entire body, including bones, blood vessels, the heart, and nerves. In particular, since bones are moved by muscles and maintain their density, when muscles become weak, bones also become weak so that osteoporosis may occur easily. In addition, when muscle mass decreases, various factors produced by muscles decrease or are oversecreted, which hinders the formation of new blood vessels and nerves and may eventually cause serious diseases such as cognitive decline. Furthermore, sarcopenia is closely related to diseases such as fatty liver, cardiac hypertrophy, pancreatic dysfunction, and diabetes.

It is important to detect and manage sarcopenia early before it progresses to a serious stage. Muscle mass decreases by about 30% in those who are in their seventies compared to their thirties or forties, but since the body weight is maintained as fat fills the space where the muscles are lost, muscle loss is often not noticed. When muscle mass decreases excessively and joint function or overall conditions deteriorate, treatment through nutrition or exercise therapies may be difficult. Currently, the diagnosis of sarcopenia is carried out by confirming muscle mass, muscle strength, and muscle function. Muscle mass is measured through methods such as dual energy X-ray absorptiometry, bioimpedance measurement, computed tomography (CT), and magnetic resonance imaging (MRI). However, since there is a low correlation between skeletal muscle mass and muscle strength, these methods have limitations as diagnostic methods. Muscle strength is measured by leg muscle strength or grip strength, and muscle function is measured by one or two of gait speed measurement, a short physical performance battery (SPPB) test, a 400-meter walk test, and a 6-minute walk test. However, since the normal muscle mass value varies depending on age, gender, height, weight, and fat mass, the criteria for determining muscle strength and muscle function are not accurate. Moreover, performing such tests is burdensome for the elderly.

Therefore, there is a need for a new diagnostic method that enables easy determination of sarcopenia with more objective criteria.

### [Disclosure]

### [Technical Problem]

The present invention was devised to solve the above-described problems, and was completed by discovering muscle tissue protein biomarkers exhibiting correlations with functional characteristics associated with sarcopenia.

Therefore, an object of the present invention is to provide a composition for diagnosing sarcopenia, comprising, as an active ingredient, an agent for measuring a protein or mRNA level of the biomarkers.

Another object of the present invention is to provide a kit for diagnosing sarcopenia, comprising an agent for measuring a protein or mRNA level of the biomarkers.

Still another object of the present invention is to provide a method for diagnosing sarcopenia and a method for providing information therefor, the method comprising a step of measuring a protein or mRNA level of the biomarkers.

However, technical problems to be solved in the present invention are not limited to the above-described problems, and other problems which are not described herein will be fully understood by those of ordinary skill in the art from the following descriptions.

### [Technical Solution]

The present invention provides a composition for diagnosing sarcopenia, including, as an active ingredient, an agent for measuring a protein or mRNA level of one or more selected from the group consisting of asporin, protein-arginine deiminase type-2 (PADI2), adenosine 5'-monophosphoramidase histidine triad nucleotide-binding protein 2 (HINT2), and hydroxysteroid dehydrogenase-like protein 2 (HSDL2).

In one embodiment of the present invention, the protein or mRNA level of asporin or PADI2 may be increased in sarcopenia patients compared to normal people, but is not limited thereto.

In another embodiment of the present invention, the protein or mRNA level of adenosine 5'-monophosphoramidase HINT2 or HSDL2 may be decreased in sarcopenia patients compared to normal people, but is not limited thereto.

In yet another embodiment of the present invention, the composition may further include an agent for measuring a protein or mRNA level of one or more selected from the group consisting of aldehyde dehydrogenase 1A1, synaptogyrin-2, troponin T, pyruvate carboxylase, mitochondrial, annexin A3, heterogeneous nuclear ribonucleoproteins C1/C2, ribosome-binding protein 1, Ig gamma-3 chain C region, ornithine aminotransferase (mitochondrial), four and a half LIM domains protein 3, threonine--tRNA ligase 1 (cytoplasmic), striatin-3, protein unc-45 homolog B, heat shock 70 kDa protein 4, long-chain specific acyl-CoA dehydrogenase (mitochondrial), peptidyl-prolyl *cis-trans* isomerase D, actin-binding LIM protein 1, adenosine monophosphate (AMP) deaminase 3, delta(3,5)-delta(2,4)-dienoyl-CoA isomerase (mitochondrial), adenosine triphosphate (ATP)-binding cassette sub-family C member 9, sorting nexin-3, serine/threonine-protein phosphatase phosphoglycerate mutase family 5 (PGAM5) (mitochondrial), eukaryotic peptide chain release factor guanosine triphosphate (GTP)-binding subunit ERF3A, dual specificity mitogen-activated protein kinase kinase 3, long-chain fatty acid transport protein 1, valacyclovir hydrolase, myozenin-3, [pyruvate dehydrogenase (acetyl-transferring)] kinase isozyme 4 (mitochondrial), and nicotinamide adenine dinucleotide phosphate hydrogen (NADPH)-cytochrome P450 reductase, but is not limited thereto.

In yet another embodiment of the present invention, the agent for measuring a protein level may be an antibody or aptamer specific to the protein, but is not limited thereto.

In yet another embodiment of the present invention, the agent for measuring an mRNA expression level may be a primer set or probe specifically binding to the mRNA, but is not limited thereto.

In yet another embodiment of the present invention, the protein or mRNA level may be a level in one or more selected from the group consisting of blood, whole blood, plasma, lymph, urine, saliva, tissue, muscle tissue, cells, organs, bone marrow, fine needle aspiration samples, core needle biopsy samples, and vacuum aspiration biopsy samples, but is not limited thereto.

Additionally, the present invention provides a kit for diagnosing sarcopenia, including the composition according to the present invention.

Additionally, the present invention provides a method for diagnosing sarcopenia and/or a method for providing information for diagnosing sarcopenia, comprising the steps of:
(S1) a step of measuring a protein or mRNA level of one or more selected from the group consisting of asporin, PADI2, adenosine 5'-monophosphoramidase HINT2, and HSDL2 in a biological sample isolated from a subject; and
(S2) a step of comparing the protein or mRNA level of the subject measured in Step (S1) with a control group.

In one embodiment of the present invention, the method may further include a step of determining that the subject is suffering from sarcopenia or is at risk of developing sarcopenia when the protein or mRNA level of asporin or PADI2 measured in Step (S1) is higher than that of the control group, but is not limited thereto.

In another embodiment of the present invention, the method may further include a step of determining that the subject is suffering from sarcopenia or is at risk of developing sarcopenia when the protein or mRNA level of adenosine 5'-monophosphoramidase HINT2 or HSDL2 measured in Step (S1) is lower than that of the control group, but is not limited thereto.

In yet another embodiment of the present invention, the method may further include a step of measuring a protein or mRNA level of one or more selected from the group consisting of aldehyde dehydrogenase 1A1, synaptogyrin-2, troponin T, pyruvate carboxylase, mitochondrial, annexin A3, heterogeneous nuclear ribonucleoproteins C1/C2, ribosome-binding protein 1, Ig gamma-3 chain C region, ornithine aminotransferase (mitochondrial), four and a half LIM domains protein 3, threonine--tRNA ligase 1 (cytoplasmic), striatin-3, protein unc-45 homolog B, heat shock 70 kDa protein 4, long-chain specific acyl-CoA dehydrogenase (mitochondrial), peptidyl-prolyl *cis-trans* isomerase D, actin-binding LIM protein 1, adenosine monophosphate (AMP) deaminase 3, delta(3,5)-delta(2,4)-dienoyl-CoA isomerase (mitochondrial), adenosine triphosphate (ATP)-binding cassette sub-family C member 9, sorting nexin-3, serine/threonine-protein phosphatase phosphoglycerate mutase family 5 (PGAM5) (mitochondrial), eukaryotic peptide chain release factor guanosine triphosphate (GTP)-binding subunit ERF3A, dual specificity mitogen-activated protein kinase kinase 3, long-chain fatty acid transport protein 1, valacyclovir hydrolase, myozenin-3, [pyruvate dehydrogenase (acetyl-transferring)] kinase isozyme 4 (mitochondrial), and nicotinamide adenine dinucleotide phosphate hydrogen (NADPH)-cytochrome P450 reductase in a biological sample isolated from the subject, but is not limited thereto.

In yet another embodiment of the present invention, the biological sample may be one or more selected from the group consisting of blood, whole blood, plasma, lymph, urine, saliva, tissue, muscle tissue, cells, organs, bone marrow, fine needle aspiration samples, core needle biopsy samples, and vacuum aspiration biopsy samples, but is not limited thereto.

In yet another embodiment of the present invention, the protein level may be measured by one or more methods selected from the group consisting of protein chip analysis, immunoassay, ligand binding assay, matrix-assisted laser desorption/ionization time-of-flight mass spectrometry (MALDI-TOF) analysis, surface-enhanced laser desorption/ionization time-of-flight mass spectrometry (SELDI-TOF) analysis, radioimmunoassay, radioimmunodiffusion, Ouchterlony immunodiffusion, rocket immunoelectrophoresis, tissue immunostaining, complement fixation assay, two-dimensional electrophoresis analysis, liquid chromatography-mass spectrometry (LC-MS), liquid chromatography-Mass Spectrometry/ Mass Spectrometry(or tandem mass spectrometry) (LC-MS/MS), Western blotting, enzyme-linked immunosorbent assay (ELISA), and fluorescence-activated single cell sorting (FACS), but is not limited thereto.

In yet another embodiment of the present invention, the mRNA level may be measured by one or more methods selected from the group consisting of polymerase chain reaction (PCR), RNase protection assay, Northern blotting, Southern blotting, *in situ* hybridization, DNA chips, and RNA chips, but is not limited thereto.

Furthermore, the present invention provides a use of an agent for measuring a protein or mRNA level of one or more selected from the group consisting of asporin, PADI2, adenosine 5'-monophosphoramidase HINT2, and HSDL2, for manufacturing an agent or kit for diagnosing sarcopenia.

Furthermore, the present invention provides a use of an agent for measuring a protein or mRNA level of one or more selected from the group consisting of asporin, PADI2, adenosine 5'-monophosphoramidase HINT2, and HSDL2, for diagnosing sarcopenia.

### [Advantageous Effects]

The present invention relates to a composition for diagnosing sarcopenia and the like, and was completed by discovering biomarkers enabling the diagnosis of sarcopenia with high accuracy and sensitivity through qualitative and quantitative analysis of the proteome of a sarcopenia animal model. In particular, the biomarkers according to the present invention were confirmed to have a positive or negative correlation with sarcopenia-related indicators such as muscle mass, grip strength, rotarod score, and inverted strength. Therefore, instead or in addition to conventional methods that have relied on the measurement of muscle mass and muscle function, through molecular diagnosis using the markers of the present invention, it is possible to diagnose sarcopenia, and furthermore, it is also possible to determine the severity of sarcopenia through the association of the markers with muscle function. In particular, biomarkers which show consistent correlations with sarcopenia indicators are also expected to be applicable as targets for the treatment of sarcopenia. Furthermore, since sarcopenia is a disease closely related to aging, the biomarkers according to the present invention are expected to be the basis for securing anti-aging treatment targets and the development of aging control technologies.

### [Description of Drawings]

FIGS. 1A and 1B illustrate a flowchart of the protein quantification process (FIG. 1A) and the quantification results (FIG. 1B) of muscle tissue samples obtained respectively from 10 ovariectomized model mice and 9 control mice.
FIGS. 2A and 2B show the results of a principal component analysis (PCA) (FIG. 2A) and a volcano plot (FIG. 2B) of the muscle tissue proteomic data of the ovariectomized model and the control group.
FIG. 3A is a table showing 14 proteins that were increased in the muscle tissue of the ovariectomized model compared to the control group, as identified in the volcano plot analysis.
FIG. 3B is a table showing 29 proteins that were decreased in the muscle tissue of the ovariectomized model compared to the control group, as identified in the volcano plot analysis.
FIGS. 4A to 4C show the results of gene ontology analysis of proteins increased in the muscle of the ovariectomized model. FIG. 4A illustrates the biological processes in which the proteins are involved, FIG. 4B illustrates the cellular compartments of the proteins, and FIG. 4C illustrates the molecular functions of the proteins.
FIG. 5 shows the results of correlation analysis between the quantitative information of muscle proteins and the muscle mass and functional characteristic indices of the tibialis anterior.
FIG. 6 shows the results of correlation analysis between the quantitative information of muscle proteins and the grip strength index.
FIG. 7 shows the results of correlation analysis between the quantitative information of muscle proteins and the rotarod test index.
FIG. 8 shows the results of correlation analysis between the quantitative information of muscle proteins and the duration of the inverted grip.

### [Best Modes]

The present invention relates to a method for diagnosing sarcopenia and the like, and was completed by discovering biomarkers enabling the diagnosis of sarcopenia with high accuracy and sensitivity through qualitative and quantitative analysis of the proteome of a sarcopenia animal model. Specifically, the inventors of the present invention performed qualitative and quantitative analyses on muscle proteins of a normal group and a sarcopenia animal model using high-resolution mass spectrometry, and selected biomarkers that exhibited significant differences between the groups. In addition, correlations between the biomarkers and muscle mass, as well as with muscle function indicators such as grip test index, rotarod index, and inverted grip duration, were analyzed, thereby confirming that the biomarkers have strong correlations with both muscle mass and muscle function associated with sarcopenia. Therefore, the present invention is expected to enable the diagnosis of sarcopenia with high accuracy and efficiency as a molecular diagnostic method capable of replacing conventional diagnostic methods that have relied solely on measurements of muscle mass and muscle function.

Therefore, a primary object of the present invention is to provide a composition for diagnosing sarcopenia, including, as an active ingredient, an agent for measuring a protein or mRNA level of one or more selected from the group consisting of asporin, protein-arginine deiminase type-2 (PADI2), adenosine 5'-monophosphoramidase histidine triad nucleotide-binding protein 2 (HINT2), and hydroxysteroid dehydrogenase-like protein 2 (HSDL2).

In the present invention, "sarcopenia" refers to a disease in which muscle strength and physical function decline due to a decrease in skeletal muscle mass. Sarcopenia was officially recognized as a disease by being assigned a diagnosis code in the United States under ICD-10-CM in 2016 and in Korea under KCD-8 in 2021. Patients with sarcopenia exhibit various physical dysfunctions due to decreased muscle strength, such as slower gait, reduced muscle endurance, and increased susceptibility to osteoporosis, falls, and fractures. In addition, sarcopenia patients experience reduced buffering functions of muscles for blood and hormones, leading to decreased basal metabolic rate, impaired control of chronic diseases, and a higher risk of exacerbation of diabetes and cardiovascular diseases. Skeletal muscle is important not only as a motor organ, but also influences the entire body including bones, blood vessels, the heart, and nerves; therefore, sarcopenia caused by the reduction of skeletal muscle may lead to conditions such as fatty liver, cardiac hypertrophy, pancreatic dysfunction, diabetes, and cognitive decline.

**In** the present invention, sarcopenia includes not only sarcopenia caused by aging or menopause, but also sarcopenia caused by other diseases. The other diseases may include, for example, obesity, infections caused by viruses or bacteria, diabetes, cancer, degenerative diseases, menopause, and degenerative diseases such as spinal stenosis, but are not limited thereto, as any disease capable of causing a reduction in muscle mass, decreased muscle strength, and/or diminished muscle function is encompassed.

Additionally, the sarcopenia may include muscular atrophy, disuse atrophy, spinal muscular atrophy, muscular dystrophy, myotonia, hypotonia, muscle weakness, progressive muscular atrophy, amyotrophic lateral sclerosis, spinal bulbar muscular atrophy, and myasthenia gravis, but is not limited thereto.

**In** the present invention, "diagnosis" refers to identifying the presence or characteristics of a pathological condition, and may include determining the presence of sarcopenia, the onset of sarcopenia, the likelihood of developing sarcopenia (risk of onset), or the prognosis of sarcopenia.

More specifically, the term "diagnosis" as used in the present specification includes determining a subject's susceptibility to a particular disease or condition, determining whether a subject currently has a particular disease or condition, determining the prognosis of a subject having a particular disease or condition (e.g., identifying the state of a tumor, determining the stage of a tumor, or determining the responsiveness of cancer to treatment), or therametrics (e.g., monitoring the status of a subject to provide information on therapeutic efficacy).

In the present invention, "asporin" refers to a protein encoded by the Aspn gene, and may be abbreviated as "Aspn" in the present invention to indicate the same protein. Asporin is a cartilage extracellular protein that is a member of the leucine-rich proteoglycan family. Asporin regulates cartilage formation by inhibiting gene expression induced by TGF-β1 in cartilage and may induce collagen mineralization by binding to collagen and calcium. Polymorphisms of the ASPN gene are known to be associated with osteoarthritis and intervertebral disc disease; however, no association with sarcopenia has been previously reported. The inventors of the present invention confirmed, through proteomic analysis of muscle tissue in a sarcopenia animal model, that the level of asporin was significantly increased in sarcopenic subjects compared to normal subjects, and in particular, exhibited a positive correlation with muscle mass and grip strength.

In the present invention, "PADI2 (protein-arginine deiminase type-2)" refers to a protein that induces post-translational deimination by converting arginine residues to citrulline, and is encoded by the PADI2 gene. Aberrations in PADI2 have been reported to be associated with diseases such as Alzheimer's disease, multiple sclerosis, and glaucoma; however, no association with sarcopenia has been previously reported. The inventors of the present invention confirmed, through proteomic analysis of muscle tissue in a sarcopenia animal model, that the level of PADI2 was significantly increased in sarcopenic subjects compared to normal subjects, and in particular, exhibited a positive correlation with muscle function, such as inverted grip and grip strength.

In the present invention, "adenosine 5'-monophosphoramidase HINT2 (abbreviated as HINT2)" refers to a protein having adenosine 5'-monophosphoramidase activity, and is encoded by the Hint2 gene. The protein hydrolyzes purine nucleotide phosphoramidates to generate AMP and NH₂. HINT2 is known to be associated with steroid biosynthesis and apoptosis. The inventors of the present invention confirmed, through proteomic analysis of muscle tissue in a sarcopenia animal model, that the level of HINT2 was significantly decreased in sarcopenic subjects compared to normal subjects, and exhibited a negative correlation with muscle mass and grip strength.

In the present invention, "HSDL2 (hydroxysteroid dehydrogenase-like protein 2)" refers to a protein having oxidoreductase activity, and is encoded by the Hsdl2 gene. The protein is highly expressed in the liver, kidney, prostate, testis, and ovary, and is also known as a major regulatory factor in lipid metabolism. Dysfunction of HSDL2 has been reported to be associated with glioma and ovarian cancer. The inventors of the present invention confirmed, through proteomic analysis of muscle tissue in a sarcopenia animal model, that the level of HSDL2 was significantly decreased in sarcopenic subjects compared to normal subjects, and exhibited a negative correlation with muscle mass and grip strength.

Asporin, PADI2, adenosine 5'-monophosphoramidase HINT2, and HSDL2 are proteins that were confirmed to exhibit the same type of correlation (positive or negative) in at least two out of the four representative diagnostic indicators for sarcopenia: muscle mass, grip test index, rotarod index, and inverted grip index. In particular, all of the above proteins showed a strong correlation with grip test and muscle mass. For example, the expression levels of asporin and PADI2 were significantly increased in sarcopenic subjects, and exhibited a positive correlation with muscle mass and grip strength. In addition, the expression levels of HINT2 and HSDL2 were significantly decreased in sarcopenic subjects, and may show a negative correlation with muscle mass and grip strength. Therefore, asporin and PADI2, and HINT2 and HSDL2, may exhibit opposing expression patterns in sarcopenic subjects.

Additionally, by measuring the level of one or more of asporin, PADI2, adenosine 5'-monophosphoramidase HINT2, and HSDL2, sarcopenia may be accurately diagnosed, and the severity of sarcopenia may also be determined based on the association with muscle mass and muscle function. For example, in a sarcopenia patient, the higher the protein or mRNA level of asporin or PADI2, the lower the muscle mass and muscle function may be assessed to be, thereby indicating a higher severity of sarcopenia. Alternatively, in a sarcopenia patient, the lower the protein or mRNA level of adenosine 5'-monophosphoramidase HINT2 or HSDL2, the lower the muscle mass and muscle function may be assessed to be, thereby indicating a higher severity of sarcopenia.

In one embodiment of the present invention, the composition may further include an agent for measuring a protein or mRNA level of one or more selected from the group consisting of aldehyde dehydrogenase 1A1, synaptogyrin-2, troponin T, pyruvate carboxylase, mitochondrial, annexin A3, heterogeneous nuclear ribonucleoproteins C1/C2, ribosome-binding protein 1, Ig gamma-3 chain C region, ornithine aminotransferase (mitochondrial), four and a half LIM domains protein 3, threonine--tRNA ligase 1 (cytoplasmic), striatin-3, protein unc-45 homolog B, heat shock 70 kDa protein 4, long-chain specific acyl-CoA dehydrogenase (mitochondrial), peptidyl-prolyl *cis-trans* isomerase D, actin-binding LIM protein 1, adenosine monophosphate (AMP) deaminase 3, delta(3,5)-delta(2,4)-dienoyl-CoA isomerase (mitochondrial), adenosine triphosphate (ATP)-binding cassette sub-family C member 9, sorting nexin-3, serine/threonine-protein phosphatase phosphoglycerate mutase family 5 (PGAM5) (mitochondrial), eukaryotic peptide chain release factor guanosine triphosphate (GTP)-binding subunit ERF3A, dual specificity mitogen-activated protein kinase kinase 3, long-chain fatty acid transport protein 1, valacyclovir hydrolase, myozenin-3, [pyruvate dehydrogenase (acetyl-transferring)] kinase isozyme 4 (mitochondrial), and nicotinamide adenine dinucleotide phosphate hydrogen (NADPH)-cytochrome P450 reductase. These proteins (genes) are markers that were statistically significantly increased or decreased in the sarcopenia animal model compared to normal subjects, and were confirmed to have positive or negative correlations with indicators used to assess muscle mass and muscle function, including muscle mass, grip test index, rotarod index, and inverted grip index.

In the present invention, asporin, PADI2, aldehyde dehydrogenase 1A1, synaptogyrin-2, troponin T, pyruvate carboxylase, mitochondrial, annexin A3, heterogeneous nuclear ribonucleoproteins C1/C2, ribosome-binding protein 1, Ig gamma-3 chain C region, ornithine aminotransferase (mitochondrial), four and a half LIM domains protein 3, threonine--tRNA ligase 1 (cytoplasmic), striatin-3, protein unc-45 homolog B, heat shock 70 kDa protein 4, long-chain specific acyl-CoA dehydrogenase (mitochondrial), and peptidyl-prolyl cis-trans isomerase D are characterized by being increased in sarcopenia patients compared to normal controls. In particular, the above proteins may be further increased in patients with low muscle mass or reduced muscle function.

In the present invention, adenosine 5'-monophosphoramidase HINT2, HSDL2, actin-binding LIM protein 1, AMP deaminase 3, delta(3,5)-delta(2,4)-dienoyl-CoA isomerase (mitochondrial), ATP-binding cassette sub-family C member 9, sorting nexin-3, serine/threonine-protein phosphatase PGAM5 (mitochondrial), eukaryotic peptide chain release factor GTP-binding subunit ERF3A, dual specificity mitogen-activated protein kinase kinase 3, long-chain fatty acid transport protein 1, valacyclovir hydrolase, myozenin-3, [pyruvate dehydrogenase (acetyl-transferring)] kinase isozyme 4 (mitochondrial), and NADPH-cytochrome P450 reductase are characterized by being decreased in sarcopenia patients compared to normal controls. In particular, the above proteins may be further decreased in patients with low muscle mass or reduced muscle function.

**In** the present specification, "an increased level" refers to the detection of a previously undetectable substance, or a relative increase in the detected amount compared to a normal level. For example, "increased" level may mean that the level in the experimental group is at least 1%, 2%, 3%, 4%, 5%, 10% or more, for example 5%, 10%, 20%, 30%, 40%, or 50%, 60%, 70%, 80%, 90% or more higher than that of the control group, and/or 0.5-fold, 1.1-fold, 1.2-fold, 1.4-fold, 1.6-fold, 1.8-fold or more higher. Specifically, it may refer to an increase of 1 to 1.5-fold, 1.5 to 2-fold, 2 to 2.5-fold, 2.5 to 3-fold, 3 to 3.5-fold, 3.5 to 4-fold, 4 to 4.5-fold, 4.5 to 5-fold, 5 to 5.5-fold, 5.5 to 6-fold, 6 to 6.5-fold, 6.5 to 7-fold, 7 to 7.5-fold, 7.5 to 8-fold, 8 to 8.5-fold, 8.5 to 9-fold, 9 to 9.5-fold, 9.5 to 10-fold, or 10-fold or more compared to the control group, but is not limited thereto. The meaning of the opposite term may be understood by those skilled in the art as having the opposite meaning according to the above definition.

As used in the present specification, the term "measuring" encompasses both measuring and confirming the presence (expression) of a target substance (in the present invention, the mRNA or protein of a sarcopenia biomarker gene), and measuring and confirming a change in the level (expression level) of the target substance. That is, measuring the expression level of the protein refers to measuring whether the protein is expressed (i.e., whether it is present) or measuring qualitative and/or quantitative changes in the expression level of the protein. The measurement may be performed without limitation using both qualitative and quantitative methods (analyses). The types of qualitative and quantitative methods for measuring protein levels are well known in the art, and include the experimental methods described in the present specification. The specific approaches for comparing protein levels by each method are also well known to those skilled in the art. Accordingly, the detection of the target protein refers to detecting the presence or absence of the biomarker protein, or confirming an increase (upregulation) or decrease (downregulation) in the level of the protein.

As used in the present specification, the term "analysis" may preferably refer to "measurement," wherein the qualitative analysis may refer to measuring and confirming the presence or absence of a target substance, and the quantitative analysis may refer to measuring and confirming the level (expression level) or amount of the target substance or changes therein. In the present invention, the analysis or measurement may be performed without limitation using both qualitative and quantitative methods, and preferably, quantitative measurement may be performed.

When the composition is for measuring an mRNA level, the agent for measuring the mRNA level may be a primer set or probe specifically binding to the mRNA. The composition of the present invention, which includes a primer set or probe specific to the mRNA of the biomarker genes according to the present invention, may further comprise agents necessary for known methods of detecting RNA. By using the composition of the present invention in conjunction with any known method of detecting RNA without limitation, the mRNA level of the biomarkers in a subject sample may be measured.

In the present invention, the "primer" refers to a fragment that recognizes a target gene sequence and includes a forward and reverse primer pair, and preferably, the primer pair provides analysis results with specificity and sensitivity. When the nucleotide sequence of the primer is mismatched with non-target sequences present in the sample, such that only the target gene sequence containing a complementary primer-binding site is amplified and nonspecific amplification is not induced, high specificity may be achieved.

In the present invention, the term "probe" refers to a substance capable of specifically binding to a target substance to be detected in a sample, and through such binding, it refers to a substance that allows specific detection of the presence of the target substance in the sample. There is no limitation on the type of probe, as it may be any substance conventionally used in the art, but preferably it may be peptide nucleic acid (PNA), locked nucleic acid (LNA), peptide, polypeptide, protein, RNA, or DNA, and most preferably PNA. More specifically, the probe may be a biomaterial derived from a biological source, a biomimetic thereof, or produced ex vivo, and may include, for example, enzymes, proteins, antibodies, microorganisms, animal or plant cells and organs, nerve cells, DNA, and RNA, wherein the DNA may include cDNA, genomic DNA, and oligonucleotides, the RNA may include genomic RNA, mRNA, and oligonucleotides, and examples of proteins may include antibodies, antigens, enzymes, and peptides.

Since the information of the biomarkers (proteins or genes) according to the present invention, such as amino acid sequences and nucleic acid sequences, is known, one of ordinary skill in the art may readily design a primer, probe, or antisense nucleotide that specifically binds to the gene encoding the protein based on such information. The primer or probe may be chemically synthesized using a phosphoramidite solid-phase synthesis method or any other well-known method, and includes all functional equivalents. In addition, the primer or probe may be variously modified using methods known in the art, so long as such modifications do not interfere with hybridization to mRNA. Examples of such modifications include methylation, capping, substitution with one or more analogs of natural nucleotides, and modifications between nucleotides, such as uncharged linkages (e.g., methyl phosphonate, phosphotriester, phosphoramidate, carbamate) or charged linkages (e.g., phosphorothioate, phosphorodithioate), as well as conjugation with labeling materials using fluorescent dyes or enzymes.

When the composition of the present invention is for measuring a protein level, the agent for measuring the protein level may be an antibody or aptamer specific to the protein.

As used in the present specification, the term "antibody" refers to a specific protein molecule that is directed against an antigenic epitope. For the purposes of the present invention, the antibody refers to an antibody that specifically binds to the marker protein, and includes polyclonal antibodies, monoclonal antibodies, and recombinant antibodies. In addition, any portion of a full-length antibody having antigenantibody binding capability is also included as an antibody of the present invention, and all types of immunoglobulin antibodies that specifically bind to the protein of the present invention are encompassed. For example, the antibody includes not only a full-length antibody comprising two full-length light chains and two full-length heavy chains, but also functional fragments of the antibody molecule that possess antigen-binding capability, such as Fab, F(ab'), F(ab')2, and Fv. Furthermore, the antibody of the present invention includes special antibodies such as humanized antibodies and chimeric antibodies, as well as recombinant antibodies, so long as they can specifically bind to the protein of the present invention.

As used in the present specification, the term "aptamer" refers to a singlestranded nucleic acid (DNA, RNA, or modified nucleic acid) having a stable tertiary structure and capable of specifically binding to an analyte to be detected in a sample, and may specifically detect the presence of a target protein in the sample. The aptamer may be produced by a conventional aptamer synthesis method, wherein an oligonucleotide sequence having selective and high binding affinity to the target protein of interest is determined and synthesized, and the 5' or 3' end of the oligonucleotide is modified with - SH, -COOH, -OH, or -NH₂ to allow binding to a functional group on an aptamer chip, but is not limited thereto.

The composition of the present invention may further include agents necessary for detecting the biomarker protein, and the expression level of the protein in a subject (or a biological sample obtained from the subject) may be measured using the composition by employing any known method for detecting a protein without limitation.

The composition according to the present invention may be used for analyzing a biological sample isolated from a subject in need of diagnosis. In the present invention, the term "biological sample" may include, without limitation, any sample collected from a subject for diagnosing sarcopenia or predicting the risk of its development. For example, the biological sample may be selected from the group consisting of blood, whole blood, plasma, urine, saliva, tissue, muscle tissue, cells, organs, bone marrow, fine needle aspiration samples, core needle biopsy samples, and vacuum aspiration biopsy samples. More specifically, the biological sample may be one or more selected from the group consisting of whole blood, leukocytes, peripheral blood mononuclear cells, buffy coat, plasma, serum, sputum, tears, mucus, nasal washes, nasal aspirate, breath, urine, semen, saliva, peritoneal washings, ascites, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extract, and cerebrospinal fluid. Preferably, the composition according to the present invention is for non-invasive diagnosis (e.g., liquid biopsy), and may be one or more selected from the group consisting of blood, whole blood, plasma, serum, and lymph fluid. Alternatively, the biological sample may be muscle tissue, and preferably, the tissue may be tibialis anterior, extensor digitorum longus, or gastrocnemius.

The biological sample may be pretreated prior to use for detection or diagnosis. For example, the pretreatment may include homogenization, filtration, distillation, extraction, concentration, inactivation of interfering components, or addition of reagents. The sample may be prepared to increase the detection sensitivity of the protein marker, and for example, a sample obtained from a subject may be pretreated using methods such as anion exchange chromatography, affinity chromatography, size exclusion chromatography, liquid chromatography, sequential extraction, or gel electrophoresis.

Additionally, the present invention provides a kit for diagnosing sarcopenia, including the composition according to the present invention.

In the present invention, the term "kit" refers to a diagnostic device or set capable of diagnosing sarcopenia by measuring the amount of mRNA or protein of the biomarkers according to the present invention contained in a sample, and is not limited so long as it is in a form that can measure the expression level of the genes from a biological sample isolated from a subject. For example, the kit may be an RT-PCR kit, DNA chip kit, ELISA kit, protein chip kit, rapid kit, mass spectrometry kit, or multiple reaction monitoring (MRM) kit, but is not limited thereto. Accordingly, the kit of the present invention may include not only an antibody that recognizes a target protein as a marker or a primer set or probe that recognizes mRNA as a marker, but also one or more additional component compositions, solutions, or devices suitable for the analysis method. In this case, the substance for detecting the expression level of the gene, mRNA, or protein may be applied without limitation on the number of times, and there is no restriction on the order of application of the respective substances; the application of each substance may be carried out simultaneously or sequentially.

Additionally, the kit according to the present invention may further include a manual describing the diagnostic method according to the present invention.

Still another object of the present invention is to provide a method for diagnosing sarcopenia or a method for providing information for diagnosing sarcopenia, comprising the steps of:
(S1) a step of measuring a protein or mRNA level of one or more selected from the group consisting of asporin, PADI2, adenosine 5'-monophosphoramidase HINT2, and HSDL2 in a biological sample isolated from a subject; and
(S2) a step of comparing the protein or mRNA level of the subject measured in Step (S1) with a control group.

The present invention also provides a method for providing information for determining (or analyzing) whether a subject has high susceptibility to the development of sarcopenia, the method comprising:
(a) measuring a protein or mRNA level of one or more selected from the group consisting of asporin, PADI2, adenosine 5'-monophosphoramidase HINT2, and HSDL2 in biological samples isolated from a subject suspected of having sarcopenia and from a control group; and
(b) determining that the subject has high risk of developing sarcopenia when the protein or mRNA level of asporin and/or PADI2 in the subject is increased compared to that in the control group (e.g., 1.5-fold, 2-fold, 2.5-fold, 3-fold, or more), and/or when the protein or mRNA level of adenosine 5'-monophosphoramidase HINT2 and/or HSDL2 in the subject is decreased compared to that in the control group (e.g., 1.5-fold, 2-fold, 2.5-fold, 3-fold, or more), and interpreting the result as indicating that the subject has a high susceptibility to sarcopenia.

In the present invention, the "subject" refers to an individual requiring diagnosis of sarcopenia, the possibility of developing sarcopenia, muscle mass, and/or muscle function, and the "target subject" in the present invention may be a mammal including a human, for example, selected from the group consisting of a human, rat, mouse, guinea pig, hamster, rabbit, monkey, dog, cat, cow, horse, pig, sheep, and goat, and is preferably a human, but is not limited thereto. The subject may be an individual in whom sarcopenia has developed or is suspected to have developed, and may refer to a patient who requires or is expected to require appropriate treatment for sarcopenia, but is not limited thereto.

In one another embodiment of the present invention, the method may further include a step of measuring a protein or mRNA level of one or more selected from the group consisting of aldehyde dehydrogenase 1A1, synaptogyrin-2, troponin T, pyruvate carboxylase, mitochondrial, annexin A3, heterogeneous nuclear ribonucleoproteins C1/C2, ribosome-binding protein 1, Ig gamma-3 chain C region, ornithine aminotransferase (mitochondrial), four and a half LIM domains protein 3, threonine--tRNA ligase 1 (cytoplasmic), striatin-3, protein unc-45 homolog B, heat shock 70 kDa protein 4, long-chain specific acyl-CoA dehydrogenase (mitochondrial), peptidyl-prolyl *cis-trans* isomerase D, actin-binding LIM protein 1, adenosine monophosphate (AMP) deaminase 3, delta(3,5)-delta(2,4)-dienoyl-CoA isomerase (mitochondrial), adenosine triphosphate (ATP)-binding cassette sub-family C member 9, sorting nexin-3, serine/threonine-protein phosphatase phosphoglycerate mutase family 5 (PGAM5) (mitochondrial), eukaryotic peptide chain release factor guanosine triphosphate (GTP)-binding subunit ERF3A, dual specificity mitogen-activated protein kinase kinase 3, long-chain fatty acid transport protein 1, valacyclovir hydrolase, myozenin-3, [pyruvate dehydrogenase (acetyl-transferring)] kinase isozyme 4 (mitochondrial), and nicotinamide adenine dinucleotide phosphate hydrogen (NADPH)-cytochrome P450 reductase in a biological sample isolated from the subject.

In one embodiment of the present invention, the method may further comprise a step of diagnosing sarcopenia, determining that the subject is at risk of developing sarcopenia (i.e., is likely to develop sarcopenia), determining that the muscle mass is lower than that of a normal individual, and/or determining that the muscle function is lower than that of a normal individual, when the protein or mRNA level of Asporin or PADI2 measured in Step (S1) is higher than that of the control group.

In another embodiment of the present invention, the method may further comprise a step of diagnosing sarcopenia, determining that the subject is at risk of developing sarcopenia (i.e., is likely to develop sarcopenia), determining that the muscle mass is lower than that of a normal individual, and/or determining that the muscle function is lower than that of a normal individual, when the protein or mRNA level of adenosine 5'-monophosphoramidase HINT2 or HSDL2 (hydroxysteroid dehydrogenase-like protein 2) measured in Step (S1) is lower than that of the control group.

The diagnostic method according to the present invention may have an area under the curve (AUC) of 0.7 or more, 0.75 or more, 0.8 or more, 0.85 or more, 0.9 or more, 0.93 or more, 0.95 or more, 0.96 or more, 0.97 or more, 0.98 or more, or 0.99 or more in a receiver operating characteristic (ROC) analysis. In addition, the diagnostic method according to the present invention may have a specificity and/or sensitivity of 70% or more, 75% or more, 80% or more, 85% or more, 90% or more, 93% or more, 95% or more, 96% or more, 97% or more, 98% or more, or 99% or more.

Detailed descriptions relating to the biomarkers, biological samples, and the like according to the present invention are omitted herein since they have been described above.

As used in the present invention, the term "method for providing information" refers to a method of providing information regarding the diagnosis of a disease, by analyzing a biological sample from a subject or by confirming an increase or decrease in the level of biomarkers according to the present invention to obtain information on the onset or potential risk of the disease. For example, it refers to a method of measuring the level of a biomarker according to the present invention and comparing it with a control group to provide information as to whether sarcopenia is likely to develop in the subject, whether there is a relatively high likelihood of sarcopenia, or whether sarcopenia has already developed. Through this method, it is also possible to predict the risk of sarcopenia, i.e., to identify high-risk individuals, and the method may also be used as a method for providing information related to the prevention and treatment of sarcopenia.

In the present invention, the term "control" may refer to a biological sample isolated from a normal subject or a subject suffering from sarcopenia.

In the present invention, the method for measuring a protein level is not particularly limited so long as it is a protein measurement method known in the art, and the protein level may be measured by methods such as protein chip analysis, immunoassay, ligand binding assay, matrix-assisted laser desorption/ionization time-of-flight mass spectrometry (MALDI-TOF) analysis, surface-enhanced laser desorption/ionization time-of-flight mass spectrometry (SELDI-TOF) analysis, radioimmunoassay, radioimmunodiffusion, Ouchterlony immunodiffusion, rocket immunoelectrophoresis, tissue immunostaining, complement fixation assay, two-dimensional electrophoresis analysis, liquid chromatography-mass spectrometry (LC-MS), liquid chromatography-Mass Spectrometry/ Mass Spectrometry (LC-MS/MS), Western blotting, enzyme-linked immunosorbent assay (ELISA), and fluorescence-activated cell sorting (FACS).

In the present invention, the method for measuring an mRNA level is not particularly limited so long as it is an mRNA measurement method known in the art, and the mRNA level may be measured by methods such as PCR, RNase protection assay, northern blotting, southern blotting, in situ hybridization, DNA chips, and/or RNA chips.

Additionally, the present invention may provide a diagnostic device for diagnosing sarcopenia.

The measurement unit of the diagnostic device of the present invention may measure the expression level of a protein or gene using an agent for measuring the expression level of one or more proteins or genes selected from the group consisting of asporin, PADI2 (protein-arginine deiminase type-2), adenosine 5'-monophosphoramidase HINT2, and HSDL2 (hydroxysteroid dehydrogenase-like protein 2), which are biomarkers according to the present invention, with respect to a biological sample obtained from a target subject. By using the agent in the measurement unit to confirm the expression level of the protein or gene, sarcopenia may be diagnosed, or it may be determined that the subject is at high risk of developing sarcopenia.

The diagnostic device of the present invention may further include a detection unit that predicts and outputs the presence or absence, severity, or type of sarcopenia in a subject based on the expression level of the protein or gene obtained from the measurement unit.

In the present invention, the detection unit may diagnose sarcopenia by generating and classifying information on sarcopenia according to the category of the expression level of the protein or gene obtained from the measurement unit.

Additionally, the present invention provides a method for treating sarcopenia, the method comprising the steps of:
(S1) measuring a protein or mRNA level of one or more selected from the group consisting of asporin, PADI2, adenosine 5'-monophosphoramidase HINT2, and HSDL2 in a biological sample isolated from a subject;
(S2) diagnosing the subject as having sarcopenia by comparing the protein or mRNA level of the subject measured in Step (S1) with that of a control group, wherein the subject is diagnosed with sarcopenia when the protein or mRNA level of asporin or PADI2 in the subject is increased compared to that of the control group, or when the protein or mRNA level of adenosine 5'-monophosphoramidase HINT2 or HSDL2 (hydroxysteroid dehydrogenase-like protein 2) in the subject is decreased compared to that of the control group; and
(S3) treating sarcopenia in the subject diagnosed with sarcopenia in Step (S2).

In one embodiment of the present invention, when the protein or mRNA level of asporin or PADI2 in a subject measured in Step (S1) is increased compared to that of a control group, or when the protein or mRNA level of adenosine 5'-monophosphoramidase HINT2 or HSDL2 (hydroxysteroid dehydrogenase-like protein 2) in the subject is decreased compared to that of the control group, the subject may be diagnosed with sarcopenia, and it may also be expected that muscle mass will increase or muscle function will improve upon application of a treatment method for sarcopenia conventionally used in the art.

Additionally, the present invention provides a method for responder characterization (i.e., a companion diagnostic method) for a therapeutic agent for sarcopenia, the method comprising the steps of:
(a) measuring a protein or mRNA expression level of one or more selected from the group consisting of asporin, PADI2, adenosine 5'-monophosphoramidase HINT2, and HSDL2 in a biological sample isolated from a subject; and
(b) determining the subject as a responder to the sarcopenia therapeutic agent when the protein or mRNA level of asporin or PADI2 in the subject measured in step (a) is increased compared to that of a control group, or when the protein or mRNA level of adenosine 5'-monophosphoramidase HINT2 or HSDL2 (hydroxysteroid dehydrogenase-like protein 2) in the subject is decreased compared to that of a control group,
wherein the sarcopenia therapeutic agent is one or more selected from the group consisting of Bimagrumab, Trevogrumab, Sarconeos, ARM-210, NA (cell therapy), NT-1654, AVGN7 (gene therapy), ATA 842, VB-102 (protein), peptide of follistatin, AAV gene therapy, TEI-SARM2, metformin, and vitamin D supplements.

In one embodiment of the present invention, the responder characterization method may predict that a superior therapeutic effect will be exhibited when the sarcopenia therapeutic agent is administered together with a treatment method conventionally used in the art for sarcopenia, if the protein or mRNA level of asporin or PADI2 in the subject measured in step (a) is increased compared to that of a control group, or if the protein or mRNA level of adenosine 5'-monophosphoramidase HINT2 or HSDL2 (hydroxysteroid dehydrogenase-like protein 2) in the subject is decreased compared to that of a control group.

The superior therapeutic effect refers to, for example, a greater or faster increase in muscle mass and/or improvement in muscle function compared to other sarcopenia patients. In addition, the treatment methods for sarcopenia may include dietary control, protein supplementation, rehabilitation therapy, and exercise, but are not limited thereto.

That is, the present invention provides a companion diagnostic method for treating a patient with sarcopenia. As used in the present invention, the term "companion diagnostics" refers to a biological diagnostic method used to measure a patient's response (e.g., expected recovery level) to a specific drug prescription or treatment, and personalized medicine using companion diagnostics enables safer prescriptions by reducing the risk of side effects associated with the treatment.

In the present invention, the term "treatment" refers to any act by which the target disease and associated metabolic abnormalities are improved or favorably altered, and may include methods such as chemotherapy, radiation therapy, surgical procedures, biological therapy, or administration of antibiotics. For example, the treatment may be carried out through fluid therapy, medication, plasma transfusion, dietary control, exercise, or rehabilitation therapy.

In the present invention, the term "chemotherapy" refers to the act of using a chemical substance to treat a specific disease and encompasses all drugs used in such treatment. Examples of the drug include Bimagrumab, Trevogrumab, Sarconeos, ARM-210, NA (cell therapy), NT-1654, AVGN7 (gene therapy), ATA 842, VB-102 (protein), peptide of follistatin, AAV gene therapy, TEI-SARM2, metformin, and vitamin D supplements.

In the present invention, the term "surgical procedure" encompasses any therapeutic or diagnostic intervention involving the physical act of the hand or the methodological use of the hand in combination with an instrument on the body of a subject in order to achieve a curative, therapeutic, or diagnostic effect.

In the present invention, the term "biological therapy" refers to a treatment method that directly or indirectly utilizes the human immune system by using a biological preparation containing a substance derived from a living organism or a substance produced using a living organism. The biological preparation includes vaccines, allergens, antigens, hormones, cytokines, enzymes, blood and plasma, immune sera, monoclonal antibodies, fermentation products, antitoxins, and laboratory diagnostic agents, which cannot have their potency and stability evaluated solely by physical or chemical tests.

In particular, the biomarkers according to the present invention are proteins having a strong correlation with sarcopenia and have potential as therapeutic targets. Therefore, for the treatment of sarcopenia, an expression inhibitor or activity inhibitor of asporin and/or PADI2 may be used, or an expression enhancer or activator of adenosine 5'-monophosphoramidase HINT2 and/or HSDL2 may be used.

### [Modes of the Invention]

Hereinafter, preferred examples are presented to help understand the present invention. However, the following examples are provided only to help understand the present invention more easily, and the contents of the present invention are not limited by the following examples.

### [Examples]

### Example 1. Production of sarcopenia animal model using ovariectomy

In this example, sarcopenia was induced in mice through ovariectomy to produce a sarcopenia animal model. Specifically, 7-week-old C57BL/6 female mice were purchased, stabilized under specific pathogen-free conditions for two weeks, and bilateral ovariectomy or sham surgery was performed at nine weeks of age. Two months after surgery, the mice were sacrificed, and the blood and the tibialis anterior, extensor digitorum longus, and gastrocnemius muscles were collected. A quantitative proteomic analysis was performed on the tibialis anterior tissue. The characteristics of the mice two months after surgery for proteomic studies are shown in Table 1 below.

**[Table 1]**

| | 2 months after surgery | | | |
|---|---|---|---|---|
| | Sham (n = 9) | OVX (n = 10) | Δ change | P |
| Body weight (g) | 23.0 ± 1.2 | 30.1 ± 2.5 | 30.9% | <0.001 |
| BMD (g/cm3) | 0.074 ± 0.003 | 0.068 ± 0.003 | -9.5% | <0.001 |
| Fat mass (g) | 1.08 ± 0.45 | 4.75 ± 1.12 | 339.8% | <0.001 |
| Lean mass (g) | 18.8 ± 1.0 | 23.3 ± 1.6 | 23.9% | <0.001 |
| Sarcopenic index (%) | | | | |
| Soleus | 0.037 ± 0.004 | 0.030 ± 0.007 | -18.9% | 0.027 |
| Tibialis anterior | 0.173 ± 0.029 | 0.129 ± 0.013 | -25.4% | <0.001 |
| Extensor digitorum longus | 0.041 ± 0.007 | 0.026 ± 0.012 | -36.6% | 0.005 |
| Gastrocnemius | 0.532 ± 0.035 | 0.400 ± 0.020 | -24.8% | <0.001 |
| Grip strength (g/g body weight) | 6.91 ± 0.54 | 5.28 ± 0.46 | -23.6% | <0.001 |
| Latency time to fall (s) | 85.3 ± 24.5 | 54.9 ± 8.7 | -35.6% | 0.006 |
| Grid hanging time (s) | 66.8 ± 63.1 | 8.6 ± 3.3 | -87.1% | 0.024 |

### Example 2. Sample preparation for proteomic analysis

The tibialis anterior muscle tissue was lysed in a 100 µL of lysis buffer containing 5% sodium dodecyl sulfate and 50 mM triethylammonium bicarbonate (pH 7.55, Thermo Fisher Scientific). Then, lysis was performed using the adaptive focused acoustics technique, and samples were subjected to S-Trap-based trypsin digestion according to the manufacturer's instructions, except that a trypsin/LysC mixture (Promega, Madison, WI, USA) was used. Dried peptides were reconstituted in 0.1% formic acid, and the absorbance was measured at 205 nm in a NanoDrop One spectrophotometer (Thermo Fisher Scientific) to determine peptide concentration. From each sample, 4 µg of peptides were allocated to individual analyses and dried. All remaining peptides were combined for the generation of a spectroscopic library.

### Example 3. Mass spectrometry for quantitative proteomics

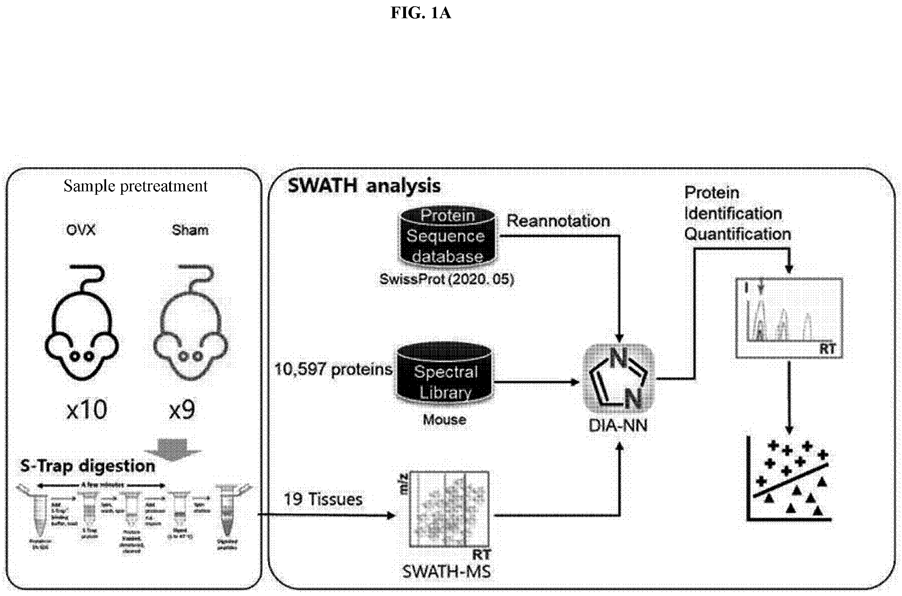

Individual muscle peptide samples were analyzed using a SCIEX TripleTOF 5600+ system mass spectrometer with the following LC-MS/MS settings: For LC separation on Ekspert^{™} NanoLC 425 (Eksigent, Dublin, CA), an Eksigent micro trap cartridge (ChromXP C18CL, 5 µm, 120 Å) was used as a trap column, an Eksigent column (C18-CL, 0.3 × 150 mm, 3 µm particle size, 120 Å pore size) was used as an analytical column, and the column temperature was maintained at 40 °C. The samples were loaded onto the trap column at a flow rate of 10 µL/min using 100% eluent A (0.1% formic acid). After ten minutes, the peptides were separated at 5 µL/min in 87 minute increments (eluent B was held at 3% to 25% for 68 min, held at 25% to 35% for 5 min, held at 35% to 80% for 2 min, held at 80% for 3 min, held at 80% to 3% for one minute, and held at 3% for 8 min) using eluent A and eluent B (0.1% formic acid in 100% acetonitrile). Mass spectrometry was carried out in the sequential window acquisition of all theoretical mass spectra (SWATH) mode with 100 variable windows according to the SCIEX technical note. The SWATH parameters were set as follows: lower limit m/z 400; upper limit m/z 1250; window overlap (Da) 1.0; collision energy spread (CES) was set to 5 for small windows, 8 for large windows, and 10 for the largest windows. MS2 spectra were obtained in the highsensitivity mode over the range of 100 to 1500 m/z for 2.5 microseconds, with a total cycle time of 2.8 seconds. FIG. 1A shows the muscle protein analysis process using mass spectrometry.

### Example 4. SWATH data analysis

SWATH-MS data for individual samples was processed using DIA-NN 1.7.10 with an in-house urine proteome spectral library. Spectra were searched for with default settings, but the m/z range was 400 to 1250 for precursors and 100 to 1500 for fragment ions. Protein quantification values were obtained using the MaxLFQ algorithm (PMID: 24942700) implemented in the Diann R package (https://github.com/vdemichev/diann-rpackage). Protein intensity values were filtered for precursors and protein groups passing a q-value higher than or equal to 0.01.

### Example 5. Statistical analysis

The free software Perseus (version 1.6.14.0) was used for differential analysis of protein intensities between samples. The values of normalized protein intensities were converted to log2 scale. Individual sample types were grouped, and at least 90% of the samples were required to be in at least one group. Missing values were replaced with random numbers derived from a normal distribution using default parameters (width: 0.3, downshift: 1.8). To determine statistically significant differences between samples, a T-test was performed using the Benjamini-Hochberg false discovery rate (FDR) (cutoff 0.05). Visualization using volcano plots and box plots was performed using the InstantClue software.

### [Experimental Examples]

### Experimental Example 1. SWATH analysis for identifying sarcopenia-related biomarkers

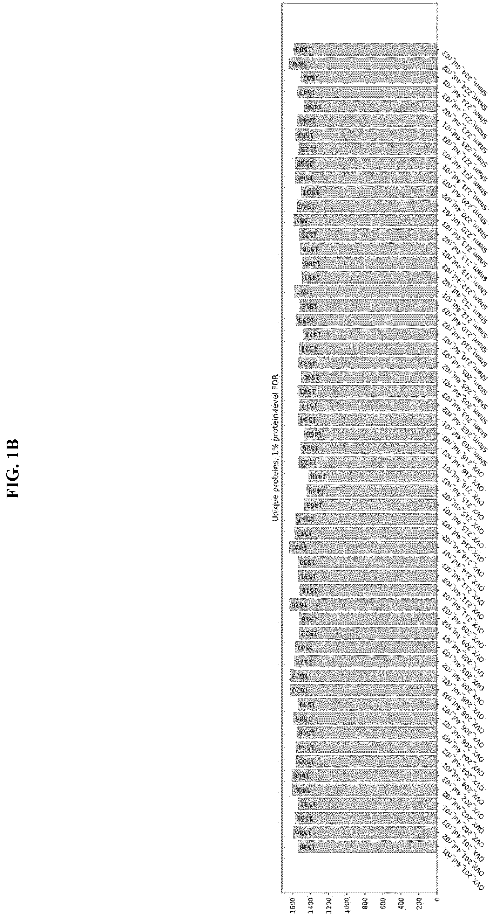

In order to select sarcopenia-related biomarker candidates, proteins were extracted from 10 ovariectomized mouse muscle tissues and 9 control mouse muscle tissues using the method described in the above examples, and protein qualitative and quantitative analyses were performed by SWATH-MS. For the obtained peptide spectra quantitative and qualitative information was extracted using DIA-NN, and a total of 2,086 proteins were quantified as shown in FIG. 1B.

### Experimental Example 2. Confirmation of the characteristics of the muscle proteins of the sarcopenia model

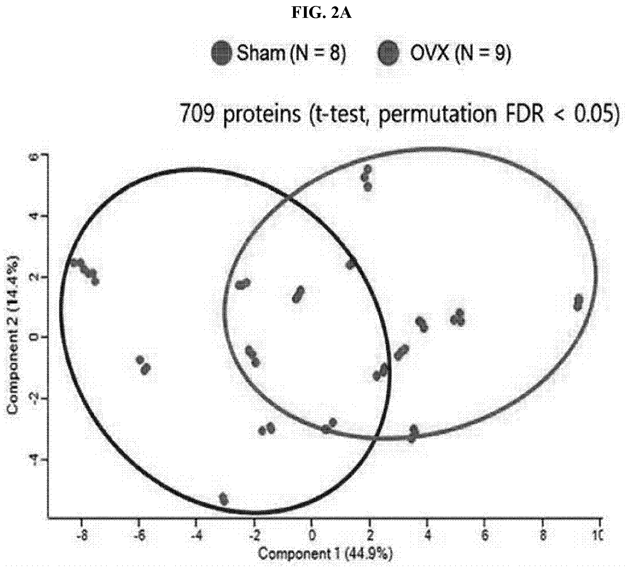

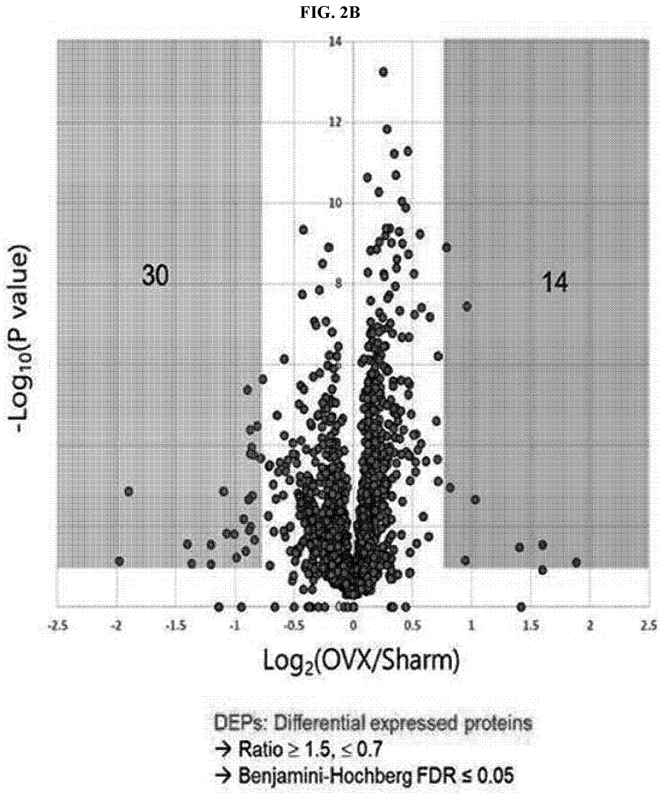

As a result of performing a principal component analysis after removing one outlier from the control group and one outlier from the ovariectomy group, it was confirmed that the ovariectomy model group and the control group were well divided, as shown in FIG. 2A. In addition, as a result of drawing a volcano plot using the fold change and P value for the quantitative values of the proteins based on the control group, it was confirmed that 14 proteins increased and 30 proteins decreased in the muscle tissue of the ovariectomy model, as shown in FIG. 2B.

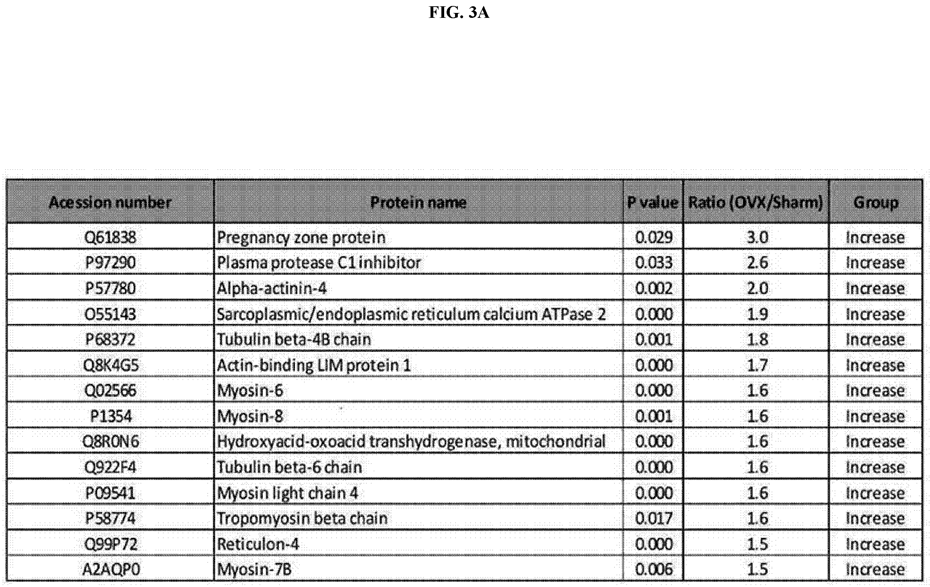

FIG. 3A shows the 14 proteins that increased in the muscle tissue of the ovariectomized model compared to the control group in the volcano plot analysis. It was found that the pregnancy zone protein increased 3-fold in the ovariectomized model compared to the control group, and the plasma protease C1 inhibitor increased 2.6-fold.

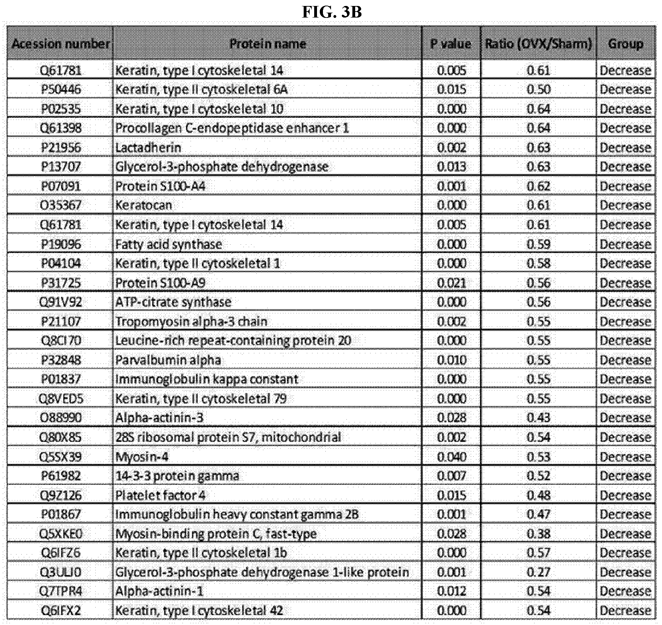

FIG. 3B shows the 29 proteins that decreased in the muscle tissue of the ovariectomized model compared to the control group in the volcano plot analysis. It was found that the myosin-binding protein C, fast-type decreased 0.38-fold in the muscle of the ovariectomized model compared to the control group, and platelet factor 4 decreased 0.48-fold.

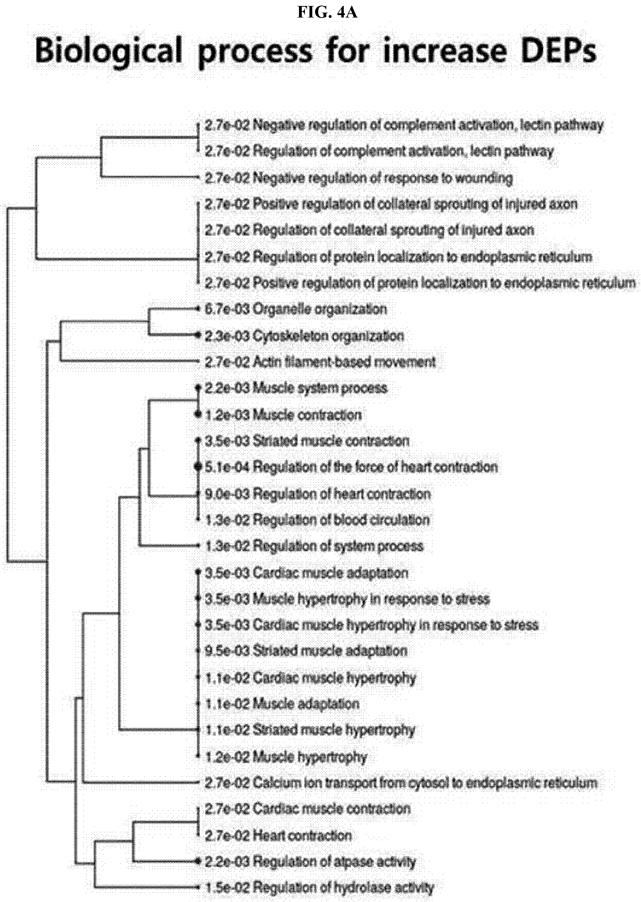

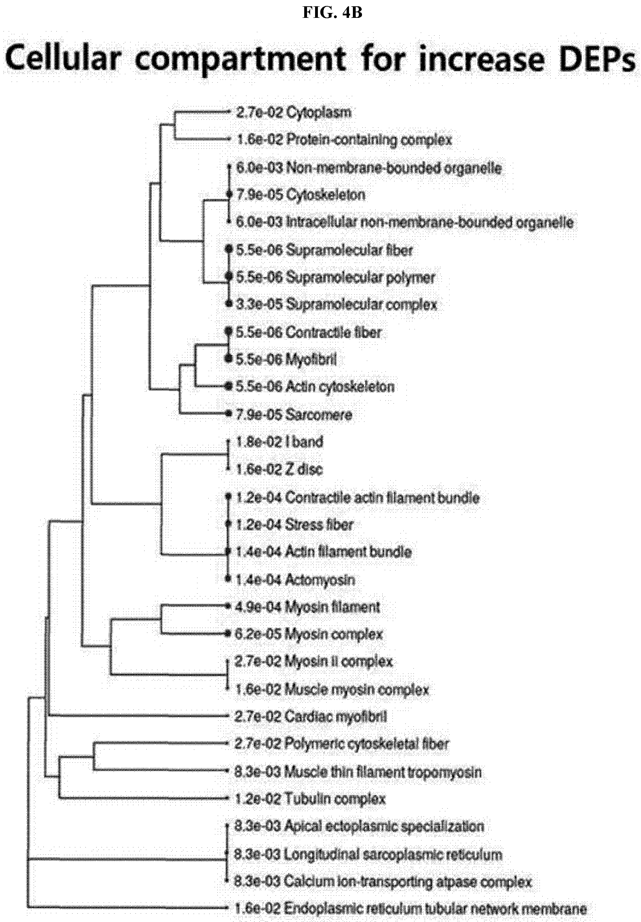

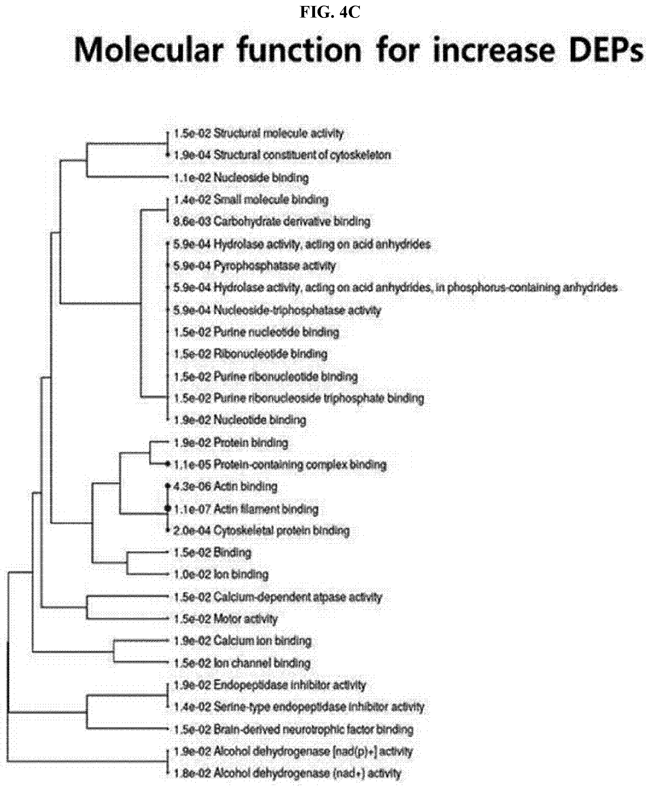

As a result of performing gene ontology analysis on the proteins increased in the muscle tissue of the ovariectomized model compared to the control group in the volcano plot analysis, the proteins were identified as proteins involved in muscle contraction and regulating injured axons (FIG. 4A). In addition, as a result of analyzing the cellular compartments of the proteins, most of them were found to have cytological locations related to contractile muscles and supramolecular fibers (FIG. 4B). From the above-described results, it can be expected that when sarcopenia is induced by ovariectomy, proteins for the tibialis anterior muscle increase more than those for other muscles, resulting in sarcopenia and functional decrease in the muscles. In addition, as a result of confirming the molecular functions of these proteins, it was found that they have actin-binding and action filament-binding functions (FIG. 4C).

### Experimental Example 3. Identification of muscle proteins correlated with muscle mass

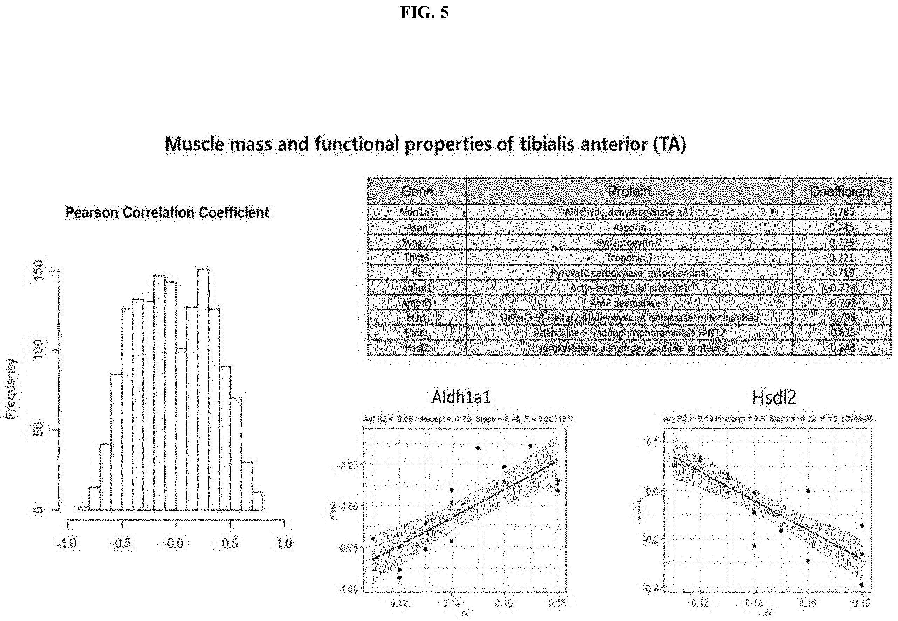

The correlation between the quantitative information of 1,527 proteins that were confirmed quantitatively by performing missing value replacement and the amount of the tibialis anterior muscle was analyzed to confirm the Pearson correlation coefficients. As a result, as shown in FIG. 5, it was found that Aldh1a1, Aspn, Syngr2, Tnnt3, and Pc proteins have a positive correlation with muscle mass. In addition, it was found that Ablim1, Ampd3, Ech1, Hint2, and Hsdl2 proteins have a negative correlation with muscle mass.

### Experimental Example 4. Identification of muscle proteins correlated with grip test index

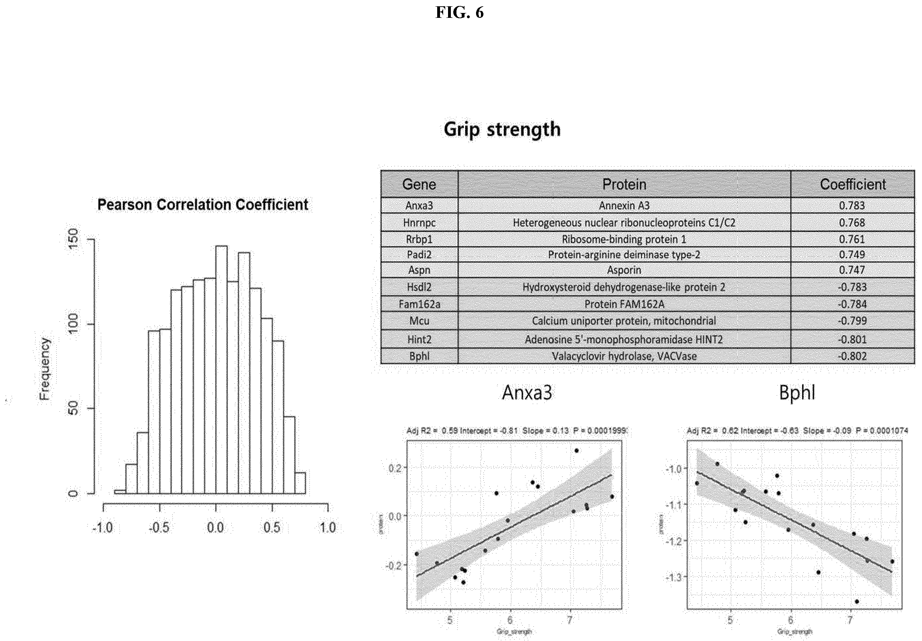

The correlation between the grip test index, which an index related to muscle function, and the amount of muscle proteins was analyzed to confirm the Pearson correlation coefficients. As a result, as shown in FIG. 6, Anxa3, Hnrnpc, Rrbp1, Padi2, and Aspn exhibited a positive correlation with muscle strength. In addition, Hsdl2, Fam162a, Mcu, Hint2, and Bphl exhibited a negative correlation with muscle strength. These results suggest that muscle strength may be represented through the quantitative relationships of intramuscular proteins.

### Experimental Example 5. Identification of muscle proteins correlated with rotarod test index

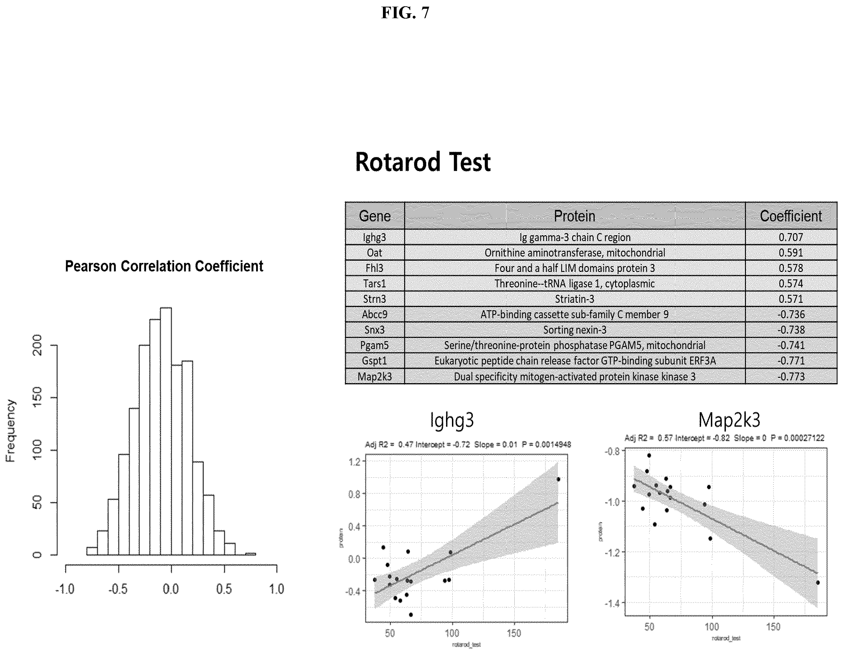

The correlation between quantitative information of muscle proteins and the rotarod test index, another type of muscle function index, was analyzed to confirm the Pearson correlation coefficients. As a result, as shown in FIG. 7, Ighg3, Oat, Fhl3, Tars1, and Strn3 proteins exhibited a positive correlation with the rotarod test results. Abcc9, Snx3, Pgam5, Gspt1, and Map2k3 exhibited a negative correlation with the rotarod test results.

### Experimental Example 6. Identification of muscle proteins exhibiting correlation with inverted grip duration

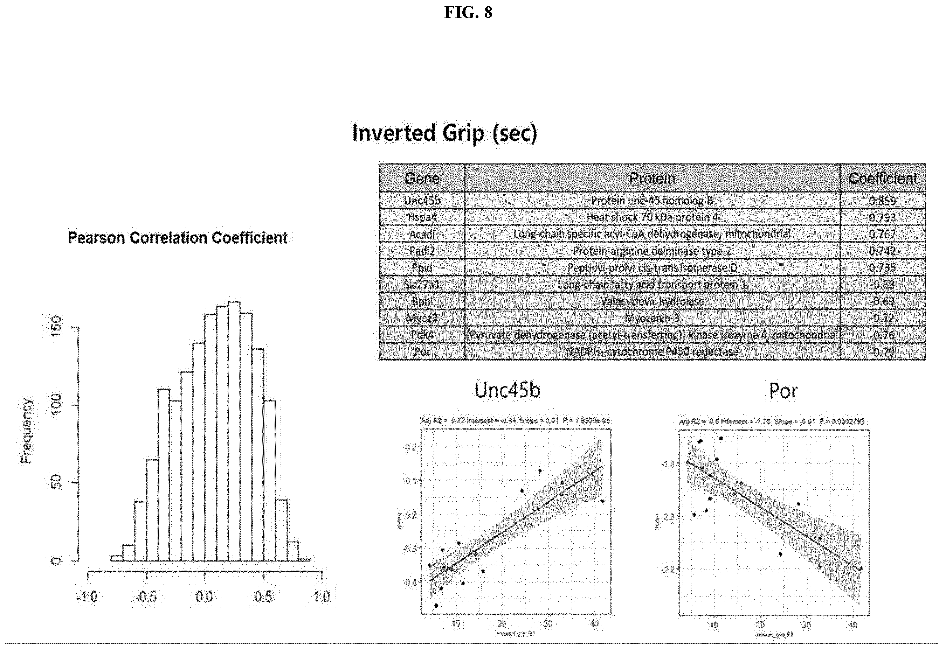

The correlation between inverted grip duration, another indicator of muscle function, and quantitative information of muscle proteins was analyzed to confirm the Pearson correlation coefficients. As a result, as shown in FIG. 8, Unc45b, Hspa4, Acadl, Padi2, and Ppid exhibited a very strong positive correlation with inverted grip duration. Unc45b showed a Pearson correlation index of 0.859 with inverted grip duration. In addition, quantitative information of Slc27a1, Bphl, Myoz3, Pdk4, and Por proteins showed a strong negative correlation with inverted grip duration.

The description of the present invention presented above is for illustrative purposes, and it should be understood that one of ordinary skill in the art to which the present invention pertains can easily make modifications into other specific forms without changing the technical ideas or essential features of the present invention. Therefore, the above-described embodiments should be understood in all respects as illustrative and not restrictive.

### [Industrial Applicability]

The present invention relates to a composition for diagnosing sarcopenia, and was completed by discovering biomarkers capable of diagnosing sarcopenia with high accuracy and sensitivity through qualitative and quantitative analysis of the proteome of a sarcopenia animal model. In particular, the biomarkers according to the present invention were confirmed to have a positive or negative correlation with sarcopenia-related indicators such as muscle mass, grip strength, rotarod index, and inverted strength. Therefore, instead of or in addition to conventional methods that have relied on measuring muscle mass and muscle function, sarcopenia may be diagnosed through molecular diagnosis using the markers of the present invention, and furthermore, the severity of sarcopenia may also be determined based on the association between the markers and muscle function. In particular, biomarkers that show consistent correlations with sarcopenia indicators are expected to be useful as targets for the treatment of sarcopenia. Furthermore, since sarcopenia is a disease closely associated with aging, the biomarkers according to the present invention are expected to serve as a foundation for securing anti-aging therapeutic targets and for developing aging control technologies, and thus have industrial applicability.

## Claims

1. A composition for diagnosing sarcopenia, comprising, as an active ingredient, an agent for measuring a protein or mRNA level of one or more selected from the group consisting of asporin, protein-arginine deiminase type-2 (PADI2), adenosine 5'-monophosphoramidase histidine triad nucleotide-binding protein 2 (HINT2), and hydroxysteroid dehydrogenase-like protein 2 (HSDL2).

2. The composition of claim 1, wherein the protein or mRNA level of asporin or PADI2 is increased in sarcopenia patients compared to normal people, and the protein or mRNA level of adenosine 5'-monophosphoramidase HINT2 or HSDL2 is decreased in sarcopenia patients compared to normal people.

3. The composition of claim 1, further comprising an agent for measuring a protein or mRNA level of one or more selected from the group consisting of aldehyde dehydrogenase 1A1, synaptogyrin-2, troponin T, pyruvate carboxylase, mitochondrial, annexin A3, heterogeneous nuclear ribonucleoproteins C1/C2, ribosome-binding protein 1, Ig gamma-3 chain C region, ornithine aminotransferase (mitochondrial), four and a half LIM domains protein 3, threonine--tRNA ligase 1 (cytoplasmic), striatin-3, protein unc-45 homolog B, heat shock 70 kDa protein 4, long-chain specific acyl-CoA dehydrogenase (mitochondrial), peptidyl-prolyl *cis-trans* isomerase D, actin-binding LIM protein 1, adenosine monophosphate (AMP) deaminase 3, delta(3,5)-delta(2,4)-dienoyl-CoA isomerase (mitochondrial), adenosine triphosphate (ATP)-binding cassette sub-family C member 9, sorting nexin-3, serine/threonine-protein phosphatase phosphoglycerate mutase family 5 (PGAM5) (mitochondrial), eukaryotic peptide chain release factor guanosine triphosphate (GTP)-binding subunit ERF3A, dual specificity mitogen-activated protein kinase kinase 3, long-chain fatty acid transport protein 1, valacyclovir hydrolase, myozenin-3, [pyruvate dehydrogenase (acetyl-transferring)] kinase isozyme 4 (mitochondrial), and nicotinamide adenine dinucleotide phosphate hydrogen (NADPH)-cytochrome P450 reductase.

4. The composition of claim 1, wherein the agent for measuring a protein level is an antibody or aptamer specific to the protein, and the agent for measuring an mRNA expression level is a primer set or probe specifically binding to the mRNA.

5. The composition of claim 1, wherein the protein or mRNA level is a level in one or more selected from the group consisting of blood, whole blood, plasma, lymph, urine, saliva, tissue, muscle tissue, cells, organs, bone marrow, fine needle aspiration samples, core needle biopsy samples, and vacuum aspiration biopsy samples.

6. A kit for diagnosing sarcopenia, comprising the composition according to any one of claims 1 to 5.

7. A method for providing information for diagnosing sarcopenia, the method comprising:
(S1) a step of measuring a protein or mRNA level of one or more selected from the group consisting of asporin, PADI2, adenosine 5'-monophosphoramidase HINT2, and HSDL2 in a biological sample isolated from a subject; and
(S2) a step of comparing the protein or mRNA level of the subject measured in Step (S1) with a control group.

8. The method of claim 7, further comprising a step of determining that the subject is suffering from sarcopenia or is at risk of developing sarcopenia when the protein or mRNA level of asporin or PADI2 measured in Step (S1) is higher than that of the control group, or when the protein or mRNA level of adenosine 5'-monophosphoramidase HINT2 or HSDL2 measured in Step (S1) is lower than that of the control group.

9. The method of claim 7, further comprising a step of measuring a protein or mRNA level of one or more selected from the group consisting of aldehyde dehydrogenase 1A1, synaptogyrin-2, troponin T, pyruvate carboxylase, mitochondrial, annexin A3, heterogeneous nuclear ribonucleoproteins C1/C2, ribosome-binding protein 1, Ig gamma-3 chain C region, ornithine aminotransferase (mitochondrial), four and a half LIM domains protein 3, threonine--tRNA ligase 1 (cytoplasmic), striatin-3, protein unc-45 homolog B, heat shock 70 kDa protein 4, long-chain specific acyl-CoA dehydrogenase (mitochondrial), peptidyl-prolyl *cis-trans* isomerase D, actin-binding LIM protein 1, adenosine monophosphate (AMP) deaminase 3, delta(3,5)-delta(2,4)-dienoyl-CoA isomerase (mitochondrial), adenosine triphosphate (ATP)-binding cassette sub-family C member 9, sorting nexin-3, serine/threonine-protein phosphatase phosphoglycerate mutase family 5 (PGAM5) (mitochondrial), eukaryotic peptide chain release factor guanosine triphosphate (GTP)-binding subunit ERF3A, dual specificity mitogen-activated protein kinase kinase 3, long-chain fatty acid transport protein 1, valacyclovir hydrolase, myozenin-3, [pyruvate dehydrogenase (acetyl-transferring)] kinase isozyme 4 (mitochondrial), and nicotinamide adenine dinucleotide phosphate hydrogen (NADPH)-cytochrome P450 reductase in a biological sample isolated from the subject.

10. The method of claim 7, wherein the biological sample is one or more selected from the group consisting of blood, whole blood, plasma, lymph, urine, saliva, tissue, muscle tissue, cells, organs, bone marrow, fine needle aspiration samples, core needle biopsy samples, and vacuum aspiration biopsy samples.

11. The method of claim 7, wherein the protein level is measured by one or more methods selected from the group consisting of protein chip analysis, immunoassay, ligand binding assay, matrix-assisted laser desorption/ionization time-of-flight mass spectrometry (MALDI-TOF) analysis, surface-enhanced laser desorption/ionization time-of-flight mass spectrometry (SELDI-TOF) analysis, radioimmunoassay, radioimmunodiffusion, Ouchterlony immunodiffusion, rocket immunoelectrophoresis, tissue immunostaining, complement fixation assay, two-dimensional electrophoresis analysis, liquid chromatography-mass spectrometry (LC-MS), liquid chromatography-Mass Spectrometry/ Mass Spectrometry (LC-MS/MS), Western blotting, enzyme-linked immunosorbent assay (ELISA), and fluorescence-activated single cell sorting (FACS).

12. The method of claim 7, wherein the mRNA level is measured by one or more methods selected from the group consisting of polymerase chain reaction (PCR), RNase protection assay, Northern blotting, Southern blotting, *in situ* hybridization, DNA chips, and RNA chips.

13. A use of an agent for measuring a protein or mRNA level of one or more selected from the group consisting of asporin, PADI2, adenosine 5'-monophosphoramidase HINT2, and HSDL2, for manufacturing an agent or kit for diagnosing sarcopenia.

14. A use of an agent for measuring a protein or mRNA level of one or more selected from the group consisting of asporin, PADI2, adenosine 5'-monophosphoramidase HINT2, and HSDL2, for diagnosing sarcopenia.
